# EUROPEAN PATENT SPECIFICATION

(11) **EP 0 570 077 B1**
(45) Date of publication and mention of the grant of the patent: **31.07.1996**
(21) Application number: 93201375.8
(22) Date of filing: 13.05.1993
(51) Int. Cl.: C08F 8/00, C08G 59/28, C08G 59/32, C07D 303/24

(54) **Curable compositions comprising glycidil ethers of hydroxyalkyl carbamates**
Härtbare Zusammensetzungen, die Glycidylether von Hydroxy-Alkyl-Carbamaten enthalten
Compositions durcissables comprenant des ethers glycidyliques d'hydroalkyl carbamates

(30) Priority: 15.05.1992 US 883738
(43) Date of publication of application: 18.11.1993
(73) Proprietor: SHELL INTERNATIONALE RESEARCH MAATSCHAPPIJ B.V., NL-2596 HR Den Haag (NL)
(72) Inventor: Bauer, Ronald Sherman, Houston, Texas 77069 (US); Blank, Werner J., Wilton, Connecticut 06897 (US)

(56) References cited:
- EP-A- 0 280 815
- FR-A- 1 496 885
- US-A- 3 239 580

## Description

This invention relates to curable coating compositions comprising glycidyl ethers of hydroxyalkyl carbamates.

Coating compositions comprising urethane structures derived from hydroxyalkylcarbamates are known in the art.

US patents Nos. 4,897,435 and 4,758,632 both disclose a process for the preparation of polymers, which are self curable at elevated temperature, by reacting a hydroxyalkylcarbamate compound with a resinous compound, such as for example an epoxy resin or an acrylic resin. US patent No. 4,520,167 discloses heat curable coating compositions comprising a hydroxyalkylcarbamate compound, an amino crosslinking agent, and a resin containing reactive groups with this crosslinking agent such as for example a polyester, polyether and acrylic resins. Here the hydroxyalkylcarbamate moieties actually serve as reactive diluent, which are built in to the matrix of the cured composition at the cure stage. US patent No. 4,820,830 relates to hydroxyalkylcarbamates and an improved process for the preparation of polyurethanediols or - polyols by reacting said hydroxyalkylcarbamates with a diol or polyol or an ester.

These hydroxyalkyl carbamates can be used in coating compositions further comprising a polyester, polyether or acrylic resin and an amino resin as curing compound.

The polyurethane diols and -polyols can further be conventionally cross linked with a polyisocyanate.

The above prior art coating compositions all require heat cure and the ones which are not self curable, are typically cured with an aminoplast resin or an isocyanate as indicated above.

Isocyanates, however, are expensive and pose handling problems, and aminoplast resins require heat to cure. Accordingly, it is an object of the invention to provide a coating composition comprising carbamate structures which cures to a hard, flexible coating but which does not require the use of isocyanate or aminoplast curing agents and which cures at ambient temperature.

Therefore the invention employs a polyglycidyl ether of the general formula I 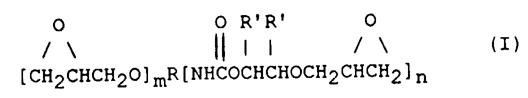 in which R is a C₁_₁₅ aliphatic or cycloaliphatic linking group; R' is H or C₁₋₃ alkyl, at least one R' being hydrogen; m is 0, 1, 2 or 3; n is 1, 2, 3 or 4; and m + n is 2, 3 or 4.

The polyglycidyl ethers of the invention are prepared by glycidating a hydroxyalkyl carbamate compound. This precursor hydroxyalkyl carbamate can be prepared by reacting a cyclic carbonate of the general formula II 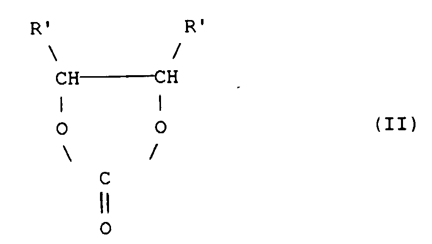 with a primary monoalkanol amine, a diamine or a polyamine of the general formula III

(HO)ₘ-R-(NH₂)ₙ (III)

in which R, m and n are as defined above. The reaction of cyclic carbonates with alkylene diamines to prepare the hydroxyalkyl carbamate precursors, has been described in EP-A-0280815. Suitable alkanolamine starting materials include monoamines such as ethanolamine, diglycolamine, 2-amino-1-butanol, 2-amino-2-methyl-1-propanol, 2-amino-2-ethyl-1,3-propane-diol and tris(hydroxymethyl)aminomethane. Suitable diamines can be represented by the general formula IV: H₂N(A)NH₂ (IV), in which A is a C₂₋₁₅ aliphatic or cycloaliphatic moiety. Preferred diamines are those in which the amine groups are separated by at least 4 carbon atoms and there is at least one methyl substituent on the alkylene moiety. Suitable aliphatic diamines include 1,2-ethylene-diamine, 1,3-propylenediamine, 1,4-pentanediamine, 1,6-hexanediamine, 2-methyl-1,5-pentanediamine, 2-ethyl-1,4-tetramethylenediamine, 2,2,6-trimethyl-1,6-hexanediamine, 2,3,6-trimethylhexamethylenediamine and 1,12-dodecanediamine. Preferred aliphatic diamines are branched aliphatic diamines such as neopentanediamine, 2-methyl-1,5-pentanediamine, 2-ethyl-1,4- tetramethylenediamine, 2,2,4-trimethyl-1,6-hexanediamine and 2,3,6-trimethyl-hexamethylenediamine. Suitable cycloaliphatic diamines include aminomethyl-cyclopentylamine, 1,2-cyclohexanediamine, 1,6-cyclohexanediamine, 6,6'-diaminocyclohexylmethane, 3-aminomethyl-3,5,5-trimethylcyclohexylamine, 3,3'-dimethyl-4,4'-diaminocyclohexylmethane and polyaminocyclohexylmethanes such as those derived from hydrogenation of aniline/-formaldehyde reaction products.

Other suitable polyamines are primary aliphatic amines containing hetero atoms such as oxygen, including such polyamines as 4,9-dioxadodecane-1,12-diamine, 3-oxapentane-1,5-diamine, 4,7,10-trioxatridecane-1,13-diamine, 3,6-dioxaoctane-1,8-diamine and the primary polyamines (including diamines) obtained from transamination of polyether polyols, predominately of polypropylene glycols. These amines are commercially available in molecular weights of 250-2000 and amine contents of 1 to 8 meq/g. Other suitable polyamines include the hydrogenation products of low molecular weight acrylonitrile oligomers and copolymers and aliphatic polyamines which can be obtained by hydrogenation of 1,3,6-tricyanohexane.

The preferred hydroxyalkyl carbamate starting materials for the glycidyl ethers of the invention are diols or polyols having a molecular weight below 1000, particularly those which can be represented by the general formula V 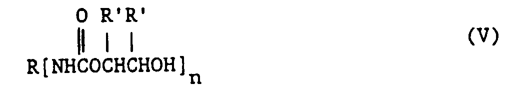 wherein R and R' are as defined above and wherein n is 2, 3 or 4.

In preparing the hydroxyalkyl carbamate precursors of the glycidyl ethers of the invention approximately equivalent amounts of the amine and the cyclic carbonate, or a slight equivalent excess of the carbonate, can be reacted at temperatures between 25 °C and 150 °C, preferably between 50 °C and 120 °C, for a time of from 30 minutes to 8 hours, preferably from 3 hours to 5 hours.

Completion of the reaction can be detected by acid titration to determine residual amine content, which should be below 3% of the original amine content, preferably below 1%. In the case of sterically hindered amines or amines on secondary or tertiary carbons, it is desirable to raise the carbonate level to assure complete reaction of the amine. Typically, the carbonate/amine ratio used is of from 1.0-1.1 for primary non-hindered amines to 1.5 for hindered amines to assure complete conversion of amine. If desired, excess carbonate can be removed after the reaction by vacuum distillation. The reaction can be carried out in a solvent such as an alcohol, ether or water. When water is present during the preparation of the carbamates, removal of water and reduction of the amine content to less than 0.5% by weight of the reaction mixture will generally take about 2 hours. If water is employed as solvent, the starting carbonate level should be somewhat higher, as some hydrolysis of the carbonate will occur in the presence of water.

After reaction of the amine and the cyclic carbonate, any hydroxylfunctional solvent should be removed to facilitate the subsequent glycidation by reaction with epichlorohydrin. Solvent removal can be carried out at a temperature within the range of from 100 °C to 225 °C at a pressure of 6.9 x 10⁻³ to 27.4 kPa (6.8 x 10⁻⁵ to 0.27 At) for 5 seconds to 60 minutes, preferably a temperature of from 120 to 150 °C, at pressure of 14.2 x 10⁻³ to 20.3 kPa (1.4 x 10⁻⁴ to 0.2 At) for a time of from 20 seconds to 20 minutes.

The properties of the hydroxyalkyl carbamates are dependent on starting materials, and the products vary in appearance from water white to slightly yellow, and can be solids or viscous liquids.

The viscosity is usually high at room temperature due to hydrogen bonding. Depending on the molecular weight or the content of hydrophobic groups, the hydroxyalkyl carbamates are either water-or organic solvent-soluble, most being soluble in solvents of solubility parameter within the range of from 10 to 15.

The glycidyl ethers used in the invention can be prepared by glycidating a hydroxyalkyl carbamate under conditions effective to produce a polyglycidyl ether. By "polyglycidyl" is meant that the epoxy resin product has, on average, at least 1.5 reactive epoxide groups per molecule. The present invention further relates to a process for preparing a polyglycidyl ether of a hydroxyalkyl carbamate by:
contacting a hydroxyalkyl carbamate and epichlorohydrin in a molar ratio within the range of from 1:10 to 1:30 at a temperature within the range of from 50 to 110 °C in the presence of an effective amount of a phase transfer catalyst to produce a condensate;
adding, over a time of from 2 to 5 hours, to the condensate-containing reaction mixture 1.5 to 4 moles of aqueous caustic per mole of the hydroxyalkyl carbamate while refluxing the reaction mixture under reduced pressure at a temperature within the range of from 35 to 50 °C; and
recovering a glycidyl ether of the hydroxyalkyl carbamate having an average of at least 1.5 epoxide groups per molecule.

This process involves the catalyzed phase transfer reaction of a polyhydroxyalkyl carbamate with epichlorohydrin under reduced pressure, followed by a ring-closing reaction carried out under mild reflux conditions in the presence of a base. The yields obtained are relatively high.

Suitable phase transfer catalysts include ammonium and phosphonium salts the currently preferred of which is tetrabutylammonium chloride. Preferably the hydroxyalkyl carbamate and the epichlorohydrin are contacted in the reaction mixture in a molar ratio within the range of from 1:15 to 1:25.

The reaction is initially carried out at about atmospheric pressure. The catalyst is generally present in the reaction mixture in an amount within the range of from 0.02 to 0.1 mole, preferably of from 0.03 to 0.05 moles per mole of the hydroxyalkyl carbamate.

The aqueous caustic used is preferably aqueous sodium or potassium hydroxide.

While refluxing the reaction mixture a water/epichlorohydrin azeotropic mixture is collected, and recovered epichlorohydrin is returned to the reaction. After substantial completion of the reaction, the product mixture is filtered for removal of by-product halides and excess caustic. The epichlorohydrin phase is washed with aqueous monobasic sodium phosphate and tap water, and remaining epichlorohydrin is removed under vacuum.

The glycidyl ethers of hydroxyalkyl carbamates used in the invention are useful as the resinous component of coating, laminating, structural composite, encapsulation, adhesive and molding powder formulations. For certain of these applications, the glycidyl ether will be used in combination with a curing agent. US-A-3239580 discloses diglycidyl ethers of hydroxyalkyl and hydroxy(polyalkoxy) carbamates. It further discloses the curing of these ethers with a curing agent such as ethanolamine or a polyalkylene polyamine. The cured products of glycidyl ethers of hydroxy(polyalkoxy) carbamates are also described in FR-A-1496885.

Suitable curing agents include aliphatic amines such as 1,2-diaminocyclohexane, aromatic amines such as 2,4-bis(p-aminobenzyl)-aniline, polyamides, anhydrides such as trimellitic anhydride, imidazoles such as 2-ethyl-4-methylimidazole, carboxylic acids, cationic compounds such as boron trifluoride complexes, and phenolics such as phenolic novolac resins. For UV-resistant coating formulations having improved UV-resistance, the preferred curing agents are aliphatic amines, aliphatic polyamides, Mannich bases, ketimines, oxazolines, amidoamines, polyoxyalkylenes, aliphatic anhydrides and modified polyamine resins prepared by reacting aliphatic or cycloaliphatic polyamines with compounds containing functional groups which react with the amine group, such as glycidyl ether-containing or carboxy-containing compounds.

Reaction products of polyamines with glycidyl ether group-containing compounds are known as "polyamine-epoxy adducts."

Reaction products of dicarboxylic acids and polyamines are known as "polyamide resins." The latter are usually prepared by condensation of the dicarboxylic acid with excess amounts of polyalkylene polyamines. Polyamides based on dicarboxylic acids having more than 10 carbon atoms, particularly those based on C₃₆ dicarboxylic acids, are preferred because of the water resistance and flexibility of the resulting coatings. Suitable modified polyamines are commercially available from the Pacific Anchor Chemical Corporation as Ancamide (Ancamide is a trade mark) amidoamine and polyamide resins, from Henkel Corporation as Versamid (Versamid is a trade mark) polyamide resins, and from Shell Chemical Company as EPON Curing Agent (EPON Curing Agent is a trade mark) V-15, V-25, V-40 and V-50 polyamide curing agents.

The curing agent will be added in an amount effective to cure the epoxy resin, usually an approximately stoichiometric quantity, so that there is of from 0.5 to 1.5 reactive NH group for each epoxide group present.

The invention is based on the finding that the glycidyl ethers are also useful as crosslinking agents for anhydride- or carboxyl-functional resins including acrylic and polyester resins. The presence of these glycidyl ethers in compositions that contain the latter resins improves the potlife of such compositions. US-A-3239580 and FR-A-1496885 are silent about the use of the ethers as cross-linking agents in these resins-containing compositions. Such acrylic resins are polymers or copolymers of acrylic acid, methacrylic acid and esters of these acids, and copolymers of such monomers with other ethylenically unsaturated monomers. Polymerization techniques for acrylic resins are known in the art and include free radical polymerization in a solvent and emulsion polymerization in water. Useful acrylic monomers include C₁₋₁₈ alkyl esters of acrylic or methacrylic acid.

Comonomers include unsaturated diacids such as maleic, fumaric and itaconic acid and the half esters thereof; styrene, α-methyl styrene, acrylonitrile, vinyl acetate, vinylchloride, and hydroxyl-containing monomers such as hydroxyethyl and hydroxypropyl acrylate or methacrylate, and the hydroxyalkyl half esters of maleic acid. The polyester or acrylate formulation will generally be prepared by adding the polyglycidyl ether of the invention to a solution or dispersion, including an aqueous solution or dispersion, of the polyester or acrylate so that the final formulation contains the polyglycidyl ether and the polyester or acrylate in a molar ratio within the range of from 5:1 to 1:5, preferably of from 2:1 to 1:2. The polyester or acrylate formulation can include an optional crosslinking catalyst such as an amine. Use of a diglycidyl ether of a hydroxyalkyl carbamate to crosslink carboxyl- and anhydride-functional acrylic resins is illustrated in the accompanying examples.

The final coating formulation usually includes sufficient solvent liquids to reduce the viscosity of the formulation to a level permitting application to a surface as a coating. Organic solvents such as alcohols; aliphatic, naphthenic and aromatic hydrocarbons; ethers; esters; and ketones can be employed.

Specific examples of such organic diluents include ethyl alcohol, isopropyl alcohol, butyl alcohol, the monomethyl ether of diethylene glycol, ethylene glycol of monobutyl ether, tetrahydrofuryl alcohol, ethyl glycol monomethyl ether, ethyl acetate, isopropyl acetate, butyl acetate, amyl acetate, acetone, methyl ethyl ketone, methyl isobuty ketone, diisobutyl ketone, hexane, heptane, acetone, methyl cyclohexane and xylene.

Alcohols such as butanol and isopropanol are the preferred organic solvents. For some applications, it may be desirable to apply a coating formulation which does not include an organic solvent.

The final coating formulation can include conventional coating additives such as accelerators; fillers and extenders such as silica, mica, quartz, cryolite, Portland cement, limestone, atomized alumina, barytes, talc, zinc powder, pyropyllite clay, diatomaceous earth; pigments such as titanium dioxide, red lead, basic lead silica chromate basic zinc chromate, zinc, zinc oxide, lead, barium and strontium chromates, calcium plumbate, barium metaborate, and calcium, strontium and zinc molybdates; and resin modifiers such as phenolic resins, urea resins, melamine resins, acrylic resins, polyester resins, vinyl resins, bituminous resins, and polystyrene.

The invention is illustrated by the following examples.

### Example 1

### Preparation of 2-Methyl-1,5-Pentanediamine Bis-β-hydroxyethylcarbamate

Into a suitable reaction vessel equipped with stirrer, thermometer, reflux condenser, and nitrogen inlet and outlet tubes were introduced 116 parts (1 mole) of 2-methyl-1,5-pentanediamine with agitation and heating.

The reactor was blanketed with nitrogen. The low-viscosity amine was heated to 50 'C, and 193.6 parts (2.2 moles) of ethylene carbonate were slowly fed into the reactor over about 1-3 hours. Cooling was used to maintain the exothermic reaction at about 90-100 °C. After about 4 hours, at a reactor temperature of 90-100 °C, the mixture was checked for amine content by acid titration. Product characteristics were as follows:

| Appearance | Viscous liquid |
|---|---|
| Nonvolatile, 60 min. at 110 °C, % | 95.4 |
| Hydroxyl number (solids) | 383 |
| Amine content | 0.064 meq/g |
| Equivalent weight (solids) | 146 |
| Equivalent weight (solution) | 153 |
| Viscosity, 25 °C, Pa.s (CPS) | 26.2 (26,200) |
| Flash point, closed cup, °C | > 93 |

### Example 2

### Glycidation of Product of Example 1

Into a suitable reaction vessel equipped with a stirrer, thermometer, dry-ice condenser, modified Dean-Stark trap and vacuum outlet were introduced 456.0 parts (1.5 mole) of the β-hydroxyethylcarbamate prepared as described in Example 1, 2775 parts (30.0 moles) epichlorohydrin and 23.0 parts tetrabutylammonium chloride (0.83 mole). The modified Dean-Stark trap was constructed such that an azeotrope could be collected under vacuum, the water and organic phase separated, and the organic phase returned to the reactor without interrupting the vacuum.

The reaction mixture was heated to 50 °C with stirring and held for 1 hour. The reaction mixture was cooled to about 40 °C, and a vacuum was then applied to the system to allow the mixture to reflux at about 35-50 °C. To the refluxing reaction mixture were added 416 parts (5.2 moles) of a 50 weight percent aqueous sodium hydroxide solution over a period of 200 minutes. The water/epichlorohydrin azeotropic mixture formed during the reaction was collected in the trap. The water-free epichlorohydrin was then returned to the reactor. After addition of the sodium hydroxide solution was completed, the reaction mixture was stirred for an additional hour. The product mixture was then filtered through a pad of diatomaceous earth, washed twice with 2000g of 20 weight percent aqueous monobasic sodium phosphate and twice with 2000mL of hot tap water, and then dried over magnesium sulfate. The excess epichlorohydrin was removed under vacuum. The product was a light yellow liquid having an epoxide equivalent weight of 203 (theoretical 209) and a viscosity (25 °C) of 7.86 Pa.s (78.6 poise).

### Example 3

Using an alternate glycidation technique, the β-hydroxyethylcarbamate prepared in Example 1 was reacted with epichlorohydrin in the presence of boron trifluoride catalyst. Into a suitable reactor equipped with stirrer, thermocouple, condenser, Dean-Stark trap, addition funnel and nitrogen inlet were introduced 91.2 parts (0.3 mole) of the β-hydroxyethylcarbamate and 212.8 parts of toluene. The toluene was heated to reflux and was separated from a water/toluene mixture which collected in the trap. After water was no longer accumulating in the trap, 1.26 parts (0.009 mole) of boron trifluoride was charged to the reactor, and addition of the epichlorohydrin was begun while the reaction mixture was held at a reflux temperature of about 109 °C. 20 minutes after epichlorohydrin addition was completed, 77.4 parts isopropyl alcohol were added, resulting in a drop of the reflux temperature of the reaction mixture to about 87 °C. Then 129 parts of a 20%w aqueous sodium hydroxide solution were added dropwise over a 90 minute period. The reflux temperature dropped to 79 °C after all the sodium hydroxide solution was added. After refluxing at this temperature for an additional 60 minutes, the reaction mixture was filtered and allowed to stand at room temperature overnight. The toluene and epichlorohydrin were removed under vacuum. The product was a yellow liquid having an EEW of 814 (theoretical 209).

### Example 4

### Preparation of 2,2,4-Trimethyl-1,6-hexamethylenediamine Bis -β-Hydroxyethyl-Carbamate

To 1000 parts of ethylene carbonate were slowly added 816 parts of a commercially available blend of 2,2,4-trimethyl-1,6-hexamethylenediamine and 2,3,4-trimethyl-1,6-hexamethylenediamine.

About 100 parts of methanol were added during the reaction to reduce foam. The reaction mixture was cooled and was then held at about 100-110 °C for about 10 hours. A vacuum was applied and the methanol was removed by distillation. The product was a bishydroxyethylcarbamate of the diamine having a viscosity of 4.3 Pa.s (43 poise) at 50 °C and solids content of 97.6 (60 min at 110 °C).

The product had a residual amine content of 0.14 MEQ/g.

### Example 5

### Glycidation of Product of Example 4

The reaction of Example 2 was repeated with 342.4 parts (1.0 mole) of the β-hydroxyethylcarbamate prepared as described in Example 4, 1850 parts (20 moles) epichlorohydrin, 26 parts tetrabutylammonium bromide, and 176 parts (2.2 moles) 50 weight percent aqueous caustic. The product diglycidyl ether was a light brown, crystalline solid and had an epoxide equivalent weight of 262 (theory 227).

### Example 6

### Preparation of Cycloaliphatic Bis-β-hydroxyethylcarbamate

1200 parts of ethylene carbonate were placed into a reactor equipped for cooling and agitation. To the carbonate were added 706 parts by weight of 1,2-cyclohexanediamine. The reaction was slightly exothermic and the mixture was maintained at 90-110 °C.

After 18 hours at 90-110 °C, the remaining amine was determined by titration to be 0.4 MEQ/g. The product had a solids content of 94.4 (60 min at 110 °C). The room temperature viscosity was too high to measure. The viscosity at 75 °C was 1.8 Pa.s (18 poise). At room temperature the product had a tendency to crystallize.

### Example 7

### Glycidation of Product of Example 6

The reaction of Example 2 was repeated with 307.2 parts (1.0 mole) of the β-hydroxyethylcarbamate prepared as described in Example 6, 1850 parts (20 moles) epichlorohydrin, 26 parts tetrabutylammonium bromide, and 176 parts (2.2 moles) 50 weight percent aqueous caustic. The product had an epoxide equivalent weight of 398 (theory 210).

### Example 8

### Preparation of 1,6-Hexanediamine Bis-β-hydroxyethylcarbamate

Example 1 was repeated, except that the 2-methyl-1,5-pentanediamine was replaced with 1,6-hexanediamine. The resulting hydroxyethylcarbamate crystallized on cooling and standing for several hours. The carbamate had a melting point of about 60 °C.

Recrystallization gave a product with a melting point of about 90-95 °C. The material had limited solubility in solvents and water and crystallized from these solvents.

### Example 9

### Preparation of Diglycidyl Ether of Linear Aliphatic Bis-β-hydroxyethyl-Carbamate

The reaction of Example 2 was repeated with 584 parts (2.0 moles) of the β-hydroxyethylcarbamate prepared as described in Example 8, 3700 parts (40 moles) epichlorohydrin, 57 parts (0.22 mole) tetrabutylammonium chloride and 352 parts (4.4 moles) 50 weight percent aqueous caustic. The product diglycidyl ether was a brown tacky solid and had an epoxide equivalent weight of 279.

### Example 10

### Preparation of 4-aminomethyl-1,8-diaminooctane Tris-β-hydroxyethylcarbamate

To 290 parts by weight of ethylene carbonate were added 173 parts by weight of 4-aminomethyl-1,8-diaminooctane at a temperature of 80-110 °C. The reaction mixture was maintained at 110 °C for 12 hours. For about 5 hours, 3.5 Pa (3.5 x 10⁻⁵ At; 27" Hg) of vacuum was applied to remove any volatile products. The final tris hydroxyethylcarbamate was brown in color and had a viscosity of 1.6 Pa.s (16 poise) at 75 °C. Solids content (60 min at 110 °C) was 97.3%. Residual amine content was 0.244 meq/g.

### Example 11

### Glycidation of Product of Example 10

The reaction of Example 2 was repeated with 239 parts (1.0 mole) of the β-hydroxyethylcarbamate prepared as described in Example 10, 1850 parts (20 moles) epichlorohydrin, 25 parts tetrabutylammonium bromide, and 176 parts (2.2 moles) 50 weight percent aqueous caustic. The product had an epoxide equivalent weight of 432 (theory 176).

### Example 12

### Preparation of Cycloaliphatic Bis-β-hydroxyethylcarbamate

To a reaction vessel were charged 760 parts by weight of ethylene carbonate, and the vessel was heated to 70 °C. 825 parts by weight of 4,4'-diaminocyclohexylmethane were slowly charged to the reaction vessel. The diamine consisted of a blend of trans/trans, cis/trans and cis/cis material with small amounts of 2,4'-isomer. 100g of xylene were charged to the reaction mixture during the reaction to reduce foaming and viscosity of the reaction blend. The reaction vessel was maintained for 24 hours at 100-110 °C.

Additional ethylene carbonate was added and elevated temperature was maintained for an additional 10 hours. The product mixture was vacuum stripped at 120 °C for 5 hours for removal of any xylene. The product hydroxyalkylcarbamate had a solids content of 95.3% (60 min, 110 °C) and a viscosity of 7.5 Pa.s (75 poise) at 75 °C.

### Example 13

### Glycidation of Product of Example 12

The reaction of Example 2 was repeated with 405 parts (1.0 mole) of the β-hydroxyethylcarbamate prepared as described in Example 12, 1850 parts (20 moles) epichlorohydrin, 25 parts tetrabutylammonium bromide, and 176 parts (2.2 moles) 50 weight percent aqueous caustic. The product had an epoxide equivalent weight of 1087 (theory 249).

### Example 14

### Preparation of Coating Formulation

The epoxide-functional urethane diol of Example 2 was used in a coating formulation as a crosslinking agent for a carboxyl-functional acrylic resin ("UCAR 884", trademark). This acrylic resin had an acid number of 25-30 as supplied and a nonvolatile content of 60%. Xylene/2-methoxypropanol (4:1) was used as the solvent.

The viscosity of the polymer solution was between 4 and 6 Pa.s. For comparison, a polyfunctional glycidyl derivative of d-glycidol (epoxide equivalent 185-210; viscosity 6-12.5 Pa.s at 25 °C) was used as a crosslinking agent for the acrylic resin. The formulations, which were catalyzed with chromium (III) octoate, are shown in Table 1. Each formulation was applied as a film to a Bonderite ("Bonderite" is a trade mark) 1000 substrate and cured at ambient temperature. As can be seen from the test results, the epoxide- functional urethane diol-containing formulation had longer pot life and displayed almost equal cure response as compared to the polyfunctional glycidol crosslinker-containing formulation.

### Example 15

A coating formulation as in Example 14 was prepared, except that the chromium catalyst was replaced with a triethylamine catalyst. Using ambient cure, the glycidated hydroxyalkyl carbamate prepared in Example 2 attained complete cure to a Knoop hardness of 15.9, the same hardness as a heat-cured coating post-cured at room temperature. Although the comparison formulation cured at room temperature to the same hardness as the invention epoxy resin, the cure was not complete, which was experienced by the increase of hardness achieved in the heat-cured system. In addition, the pot life of the comparison crosslinker was only 24 hours, while the pot life of the invention epoxide-functional urethane diol was more than 96 hours. Formulations and test results are shown in Table 2.

### Example 16

The room temperature cure response of the urethane epoxide prepared in Example 2 was tested in a water-borne acrylic emulsion.

This formulation was compared with that containing a commercially-available epoxy curing agent based on a diglycidyl ether of bisphenol-A. The manufacturer's formulation was followed.

As shown in Table 3, the formulation containing the invention urethane epoxide cured to an increased hardness and slightly improved solvent resistance. In addition, it was observed that the formulation containing the comparison crosslinker gelled within three weeks, while the urethane epoxide-containing formulation was still liquid after this time. The invention urethane epoxide has the further advantage of being water soluble, while the BPA-based epoxy resin requires emulsification.

### Example 17

Example 14 was repeated, except that no catalyst was added to the formulation. As shown in Table 4, the Knoop hardness and pencil hardness of the cured, uncatalyzed formulation were higher and the stability was greater, as shown by the viscosity after 24 hours.

### Example 18

### Preparation of an Anhydride-functional Acrylic Polymer

| Component | Monomer Weight % | Charge (g) |
|---|---|---|
| Butyl acrylate | 53 | 206 |
| Styrene | 30 | 112 |
| Maleic anhydride | 14 | 56 |
| Ethyl 3,3-di(t-amylperoxy)butyrate | 4 | 16 |
| Ethyl 3-ethoxypropionate | | 84 |
| Xylene | | 83 |
| Total | 100 | 557 |

The xylene was charged to a reactor and heated under nitrogen to reflux. The monomers were blended in a separate reactor and agitated until the maleic anhydride was dissolved. The free radical catalyst was charged and fed to the monomer blend during a two- to three-hour period while reflux was maintained. After monomer addition, the reflux was continued for two hours. The mixture was then cooled to 100 °C and the ethyl 3-ethoxypropionate solvent was charged.

### Example 19

The anhydride-functional acrylic resin of Example 18 was crosslinked with the urethane epoxide of Example 2. Coatings were cured at room temperature for 4 and 14 days, respectively. As shown in Table 5, the Knoop hardness developed in the coating after 4 days was about equal to that achieved from 30-min. cure at 120 °C and from 14 days at room temperature, indicating that essentially complete cure was achieved at ambient temperature. In addition, the coating was tested for resistance to acid etching with 20% sulfuric acid. A drop of the acid was placed on the test panel, which was placed on a gradient heater at 50, 60 and 70 °C. Even at an exposure temperature of 70 °C, the formulation showed no or very little visible attack. A typical automotive high solids acrylic/melamine coating tested as a standard gave significant attack at 50 °C and was completely destroyed at 70 °C.

### Example 20

A commercially-available water-borne acrylic emulsion (XC-4006 from American Cyanamid) with a solids content of 48% and an acrylic resin acid number of 110 was blended with the urethane epoxide of Example 2 at 29 and 17% levels based on the resin solids. The formulations were cured at room temperature (25 °C) for 12 days and at 150 °C for 20 minutes. The formulations gave flexible coatings with good adhesion. Results are shown in Table 6.

### Example 21

A commercially-available solvent-borne acrylic resin (XC-4005 from American Cyanamid) having an acid number of 110 was crosslinked with the urethane epoxide of Example 2. 2-Methyl-1,5-pentanediamine was used as a catalyst and as a tetra-functional amine reactant. The coating gave tack-free coatings overnight and cured to a pencil hardness of HB-F after one month. MEK double rubs were 50, which indicates good cure. Results are shown in Table 7.

### Example 22

Two samples of a commercially-available acrylic emulsion for maintenance application ("Maincote HG-54", a trademark of Rohm & Haas) were crosslinked with 6.7 and 3%, respectively, of the urethane epoxide prepared in Example 2. Cosolvents and additives were used according to the recommendation of the manufacturer. The formulations were cured at room temperature, 20 minutes at 150 °C, and then post-cured at room temperature for 14, 23 and 90 days. As shown in Table 8, Knoop hardness gradually increased at the room temperature cure from initially 1.7 to 4.6 for the 6.7% crosslinker formulation. The MEK resistance also increased from the initial 50 MEK rubs to 150 rubs. The formulation cured at 150 °C initially gave higher hardness and MEK resistance and continued to cure at room temperature. A similar trend was seen for the formulation modified at the 3% crosslinker level. Test results are shown in Table 8.

**TABLE 1**

| COATING FORMULATIONS | WT. % | CHARGE (g) | WT. % | CHARGE (g) |
|---|---|---|---|---|
| ACRYLIC RESIN | 92.09 | 166.67 | 92.09 | 166.67 |
| EPOXIDE CROSSLINKER | 6.91 | 7.50 | 0.00 | 0.00 |
| EPOXY OF EX. 2 | 0.00 | 0.00 | 6.91 | 7.50 |
| XYLENE | | 98.14 | | |
| 2-METHOXYPROPANOL | | | | 94.37 |
| n-BUTANOL | | 7.01 | | |
| CHROMIUM (II) OCTOATE | 1.00 | 1.09 | 1.00 | 1.09 |
| TOTAL | 100.00 | 280.40 | 100.00 | 269.62 |

| SOLUTION | | | | |
|---|---|---|---|---|
| SOLID (% CALC) | | 38.72 | | 40.27 |
| VISC. mPas.s | | 93 | | 128 |
| 24 HR VISC. mPa.s | | GEL | | 612 |

| CURED PROPERTIES | | | | |
|---|---|---|---|---|
| CURE (DAYS AT ROOM TEMP.) | 1 | 14 | 1 | 14 |
| FILM (micron) | 20 | 20 | 20 | 20 |
| APPEARANCE | GLOSSY | GLOSSY | GLOSSY | GLOSSY |
| KNOOP HARDNESS | 7.2 | 15.2 | 6.6 | 14.1 |
| PENCIL HARDNESS | B-HB | H-2H | B-HB | H-2H |
| IMPACT REV (IN. LB.) | 5 | 5 | 5 | 5 |
| FRONT (IN. LB.) | 5 | 30 | 5 | 30 |
| CROSSHATCH (ADH. %) | 100 | 100 | 100 | 100 |
| MEK (RUBS) | 10 | 70 | 10 | 55 |
| ELONGATION (%) | 1.5 | 1.5 | 1.5 | 1.5 |
| SALTSPRAY CREEP (mm) | | 3.0 | | 3.0 |
| CLEV HUMIDITY (500 HRS) | | 9VF | | 8M |

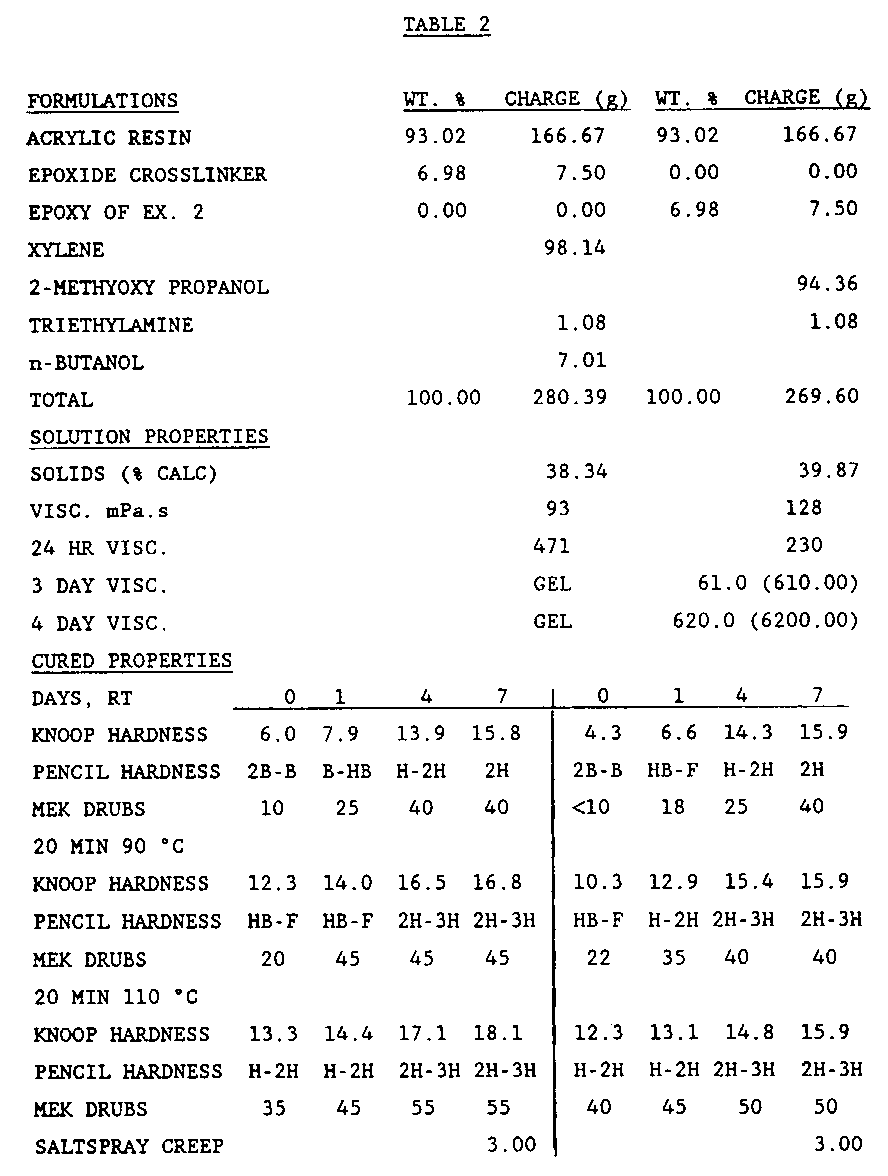

**TABLE 3**

| FORMULATIONS | WT. % | CHARGE (g) | WT. % | CHARGE (g) |
|---|---|---|---|---|
| ACRYLIC RESIN | 89.19 | 235.29 | 89.33 | 235.29 |
| EPOXY CURING AGENT | 10.81 | 21.76 | | |
| EPOXY OF EX. 2 | | | 10.67 | 11.94 |
| WATER | | 46.00 | | 46.00 |
| BUTYL CELLOSOLVE | | 12.08 | | 11.62 |
| TOTAL | 100.00 | 303.05 | | 293.24 |

| SOLUTION PROPERTIES | | | | |
|---|---|---|---|---|
| SOLID (% CALC) | | 37.00 | | 38.17 |
| VISC. mPas.s | | 98 | | 88 |
| 24 HR VISC. mPa.s | | 64 | | 110 |

| CURED PROPERTIES | | | | |
|---|---|---|---|---|
| CURE (DAYS) | 1 | 7 | 1 | 7 |
| FILM (micron) | 20 | 20 | 20 | 20 |
| APPEARANCE | GOOD | GOOD | GOOD | GOOD |
| KNOOP HARDNESS | 1.9 | 3.3 | 1.9 | 4.7 |
| PENCIL HARDNESS | HB-F | F-H | HB-F | F-H |
| IMPACT REV IN. LB. | 160 | 160 | 160 | 160 |
| FRONT IN. LB. | 160 | 160 | 160 | 160 |
| CROSSHATCH ADH. % | 100 | 100 | 100 | 100 |
| MEK DRUBS | 16 | 25 | 25 | 35 |

**TABLE 4**

| FORMULATIONS | WT. % | CHARGE (g) | WT. % | CHARGE (g) |
|---|---|---|---|---|
| ACRYLIC RESIN | 93.02 | 166.67 | 93.02 | 166.67 |
| EPOXIDE CROSSLINK AGENT | 6.98 | 7.50 | 0.00 | 0.00 |
| EPOXY OF EX. 2 | 0.00 | 0.00 | 6.98 | 7.50 |
| XYLENE | | 97.53 | | |
| 2-METHOXYPROPANOL | | | | 93.78 |
| n-BUTANOL | | 6.97 | | |
| TOTAL | 100.00 | 278.67 | 100.00 | 267.95 |

| SOLUTION PROPERTIES | | | | |
|---|---|---|---|---|
| SOLID, % CALC | | 38.50 | | 40.10 |
| VISCOSITY, 25°C, mPas.s | | 93 | | 128 |
| 24 HR VISC. mPa.s | | 447 | | 226 |

| CURED PROPERTIES | | | | |
|---|---|---|---|---|
| CURE AT ROOM TEMPERATURE (DAYS ) | | 14 | 14 | |
| FILM THICKNESS micron | | 20 | 20 | |
| APPEARANCE | | GLOSSY | GLOSSY | |
| KNOOP HARDNESS | | 14.2 | 17.9 | |
| PENCIL HARDNESS | | H-2H | 2H-3H | |
| IMPACT REV IN. LB. | | 0-20 | 0-20 | |
| FRONT IN. LB. | | 0-20 | 0-20 | |
| CROSSHATCH ADH. % | | 100 | 100 | |
| MEK DOUBLE RUBS | | 50 | 50 | |
| CONICAL MANDREL ELONGATION, | | 1.5 | 1.5 | |
| PASS, % | | | | |

**TABLE 5**

| FORMULATIONS | WT. % | CHARGE (g) |
|---|---|---|
| ANHYDRIDE-FUNCTIONAL ACRYLIC | 63.86 | 35.71 |
| EPOXY OF EX. 2 | 35.14 | 13.76 |
| 2-METHOXYPROPANOL | | 17.00 |
| TRIETHYLENE DIAMINE | 1.00 | 0.39 |
| TOTAL | 100.00 | 66.86 |

| SOLUTION PROPERTIES | | |
|---|---|---|
| SOLID (% CALC) | | 58.6 |
| INDUCTION TIME (MIN) | | 30 |
| TACK FREE | | >6 HR |
| THRU DRY | | >6 HR |
| VISCOSITY, 25°C, 0 HOURS, mPas.s | | 219 |
| VISCOSITY, 25°C, 1/2 HOURS, mPas.s | | 245 |
| CURED PROPERTIES | | |
| CURE TIME (AMBIENT) | 4 DAYS | 14 DAYS |
| INDUCTION TIME, min | 30 | 30 |
| FILM THICKNESS (micron) | 18 | 18 |
| KNOOP HARDNESS | 3.7 | 3.5 |
| PENCIL HARDNESS | 2B-B | HB-F |
| IMPACT DIR IN. LB. | 140-160 | 140-160 |
| CROSSHATCH ADH. % | 100 | 100 |
| MEK DRUBS | 25 | 25 |
| CONICAL MANDREL ELONGATION % | | 31.3 |
| SALTSPRAY, 100 CREEP mm | | 3.0 |

| PROPERTIES AFTER CURE FOR 30 MIN AT 120 °C | | |
|---|---|---|
| FILM THICKNESS (micron) | 18 | |
| KNOOP HARDNESS | 3.7 | |
| PENCIL HARDNESS | 2B-B | |
| IMPACT DIR IN. LB. | 160 | |
| CROSSHATCH ADH. % | 100.0 | |
| MEK DRUBS | 50 | |
| CONICAL MANDREL ELONGATION % | 31.3 | |
| SALTSPRAY, 150 HOURS, CREEP, mm | 3.0 | |
| ACID ETCHING, 20% SULFURIC ACID, 50/60/70°C | PASS | |

**TABLE 6**

| FORMULATIONS | WT. % | CHARGE (g) | WT. % | CHARGE (g) |
|---|---|---|---|---|
| ACRYLIC WATER | 70.7 | 100.0 | 82.8 | 100.0 |
| EMULSION | | | | |
| EPOXY OF EX. 2 | 29.3 | 19.9 | 17.2 | 10.0 |
| WATER | | 8.5 | | 4.2 |
| TOTAL | 100.0 | 128.4 | 100.0 | 114.2 |

| PROPERTIES | | | | |
|---|---|---|---|---|
| SOLID (% CALC) | | 52.9 | | 50.8 |

| CURED PROPERTIES (COLD ROLLED STEEL) | | | | |
|---|---|---|---|---|
| CURE SCHEDULE °C | 12 DAYS 25 | 20 MIN 150 | 12 DAYS 25 | 20 MIN 150 |
| FILM THICKNESS (micron) | 25 | 25 | 25 | 25 |
| APPEARANCE | GLOSSY | DULL | GLOSSY | GLOSSY |
| KNOOP HARDNESS | 1.1 | 3.0 | 1.1 | 2.0 |
| PENCIL HARDNESS | B-HB | HB-F | 3B-2B | HB-F |
| IMPACT REV IN. LB. | | 160 | | 160 |
| FRONT IN. LB. | | 160 | | 160 |
| CROSSHATCH ADH. % | 100 | 100 | 100 | 100 |
| MEK DOUBLE RUBS | 40 | 90 | 40 | 90 |
| CONICAL MANDREL 1/8" | | PASS | | PASS |

**TABLE 7**

| FORMULATIONS | WT. % | CHARGE (g) |
|---|---|---|
| ACRYLIC RESIN | 58.6 | 66.7 |
| EPOXY OF EX. 2 | 39.2 | 33.4 |
| 2-METHYL-1,5-PENTANEDIAMINE | 2.2 | 1.9 |
| 2-METHOXYPROPANOL | | 35.0 |
| TOTAL | 100.0 | 137.0 |
| SOLID % CALCULATED | | 62.3 |

| CURED PROPERTIES (COLD ROLLED STEEL) | | |
|---|---|---|
| CURE SCHEDULE, 25 °C, DAYS | 3 | 30 |
| FILM THICKNESS (micron) | 25 | 25 |
| APPEARANCE | GLOSSY | GLOSSY |
| KNOOP HARDNESS | 0.35 | 1.30 |
| PENCIL HARDNESS | <4B | HB-F |
| IMPACT REV IN. LB. | | 160 |
| FRONT IN. LB. | | 160 |
| CROSSHATCH ADH. % | 100 | 100 |
| MEK DRUBS | 20 | 50 |
| CONICAL MANDREL 1/8" | PASS | PASS |

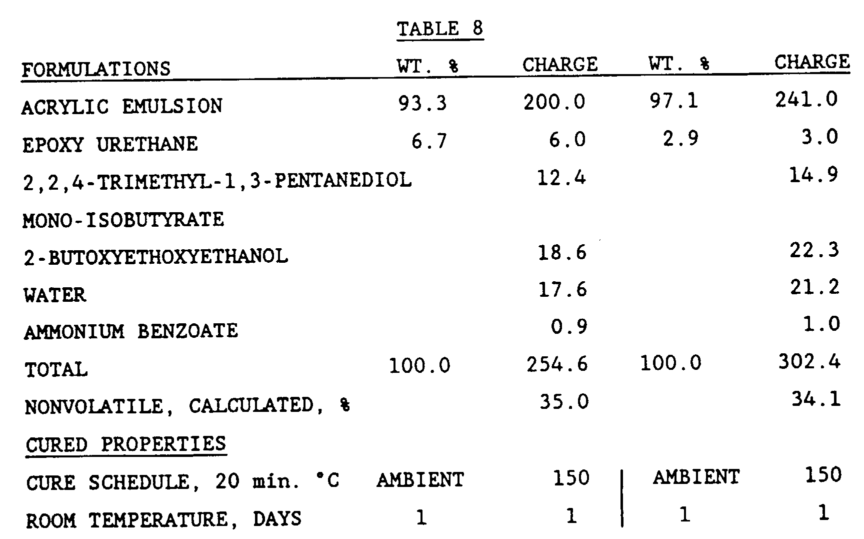

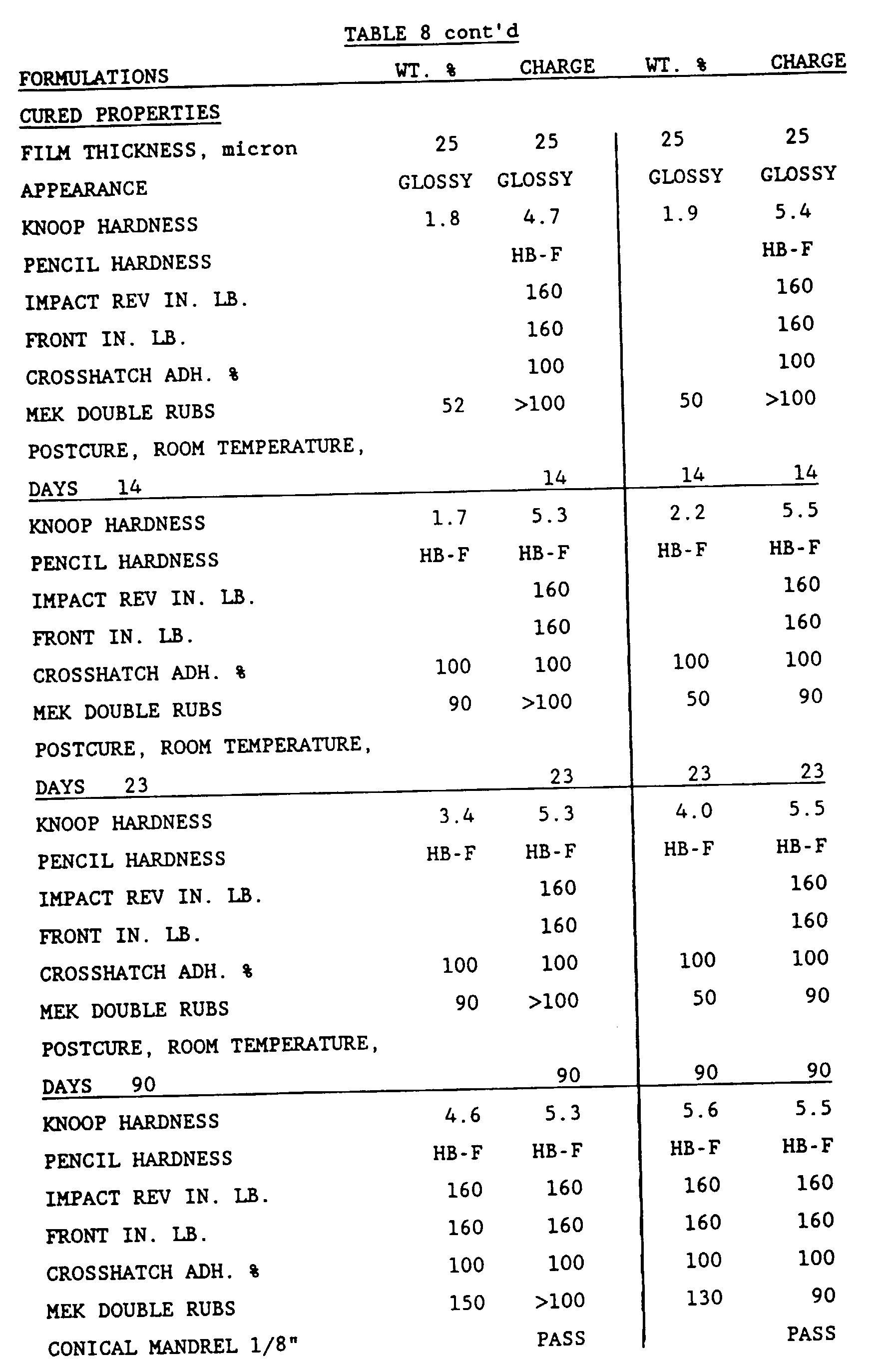

## Claims (Claims for the following Contracting State(s): BE, CH, DE, FR, GB, IT, LI, NL, SE)

1. A curable composition comprising a polyglycidyl ether of the general formula I 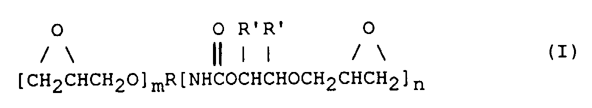 in which R is a C₁_₁₅ aliphatic or cycloaliphatic linking group;
R' is H or C₁₋₃ alkyl, at least one R' being hydrogen; m is 0, 1, 2 or 3; n is 1, 2, 3 or 4; and m + n is 2, 3 or 4, and an anhydride-or carboxyl-functional resin.

2. A composition a claimed in claim 1 wherein said resin is an acrylic resin or a polyester resin.

## Claims (Claims for the following Contracting State(s): ES)

1. A process for the preparation of a curable composition comprising mixing a polyglycidyl ether of the general formula I 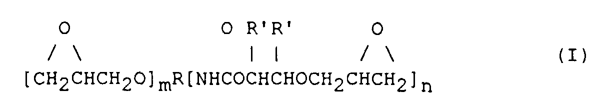 in which R is a C₁₋₁₅ aliphatic or cycloaliphatic linking group;
R' is H or C₁₋₃ alkyl, at least one R' being hydrogen; m is 0, 1, 2 or 3; n is 1, 2, 3 or 4; and m + n is 2, 3 or 4, and an anhydride-or carboxyl-functional resin.

2. A process as claimed in claim 1 wherein said resin is an acrylic resin or a polyester resin.

## Patentansprüche (Patentansprüche für folgende(n) Vertragsstaat(en): BE, CH, DE, FR, GB, IT, LI, NL, SE)

1. Härtbare Zusanmensetzung, enthaltend ein Polyglycidylether der allgemeinen Formel I 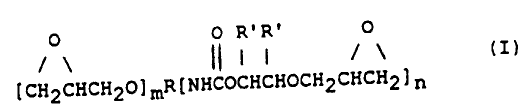 worin R für eine aliphatische oder cycloaliphatiche C₁₋₁₅-Brückengruppe und R' für H oder C₁₋₃-Alkyl, wobei mindestens ein R' Wasserstoff ist, steht, m 0, 1, 2 oder 3, n 1, 2, 3 oder 4 und m + n 2, 3 oder 4 bedeutet, und ein Harz mit Anhydrid- oder Carboxylfunktionen.

2. Zusammensetzung nach Anspruch 1, bei der es sich bei dem Harz um ein Acrylharz oder ein Polyesterharz handelt.

## Patentansprüche (Patentansprüche für folgende(n) Vertragsstaat(en): ES)

1. Verfahren zur Herstellung einer härtbaren Zusammensetzung, bei dem man ein Polyglycidylether der allgemeinen Formel I 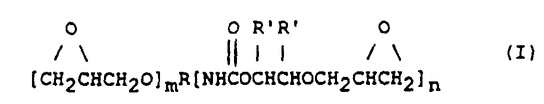 worin R für eine aliphatische oder cycloaliphatische C₁₋₁₅-Brückengruppe und R' für H oder C₁₋₃-Alkyl, wobei mindestens ein R' Wasserstoff ist, steht, m 0, 1, 2 oder 3, n 1, 2, 3 oder 4 und m + n 2, 3 oder 4 bedeutet, und ein Harz mit Anhydrid- oder Carboxylfunktionen vermischt.

2. Verfahren nach Anspruch 1, bei dem es sich bei dem Harz um ein Acrylharz oder ein Polyesterharz handelt.

## Revendications (Revendications pour l'(les) Etat(s) contractant(s) suivant(s): BE, CH, DE, FR, GB, IT, LI, NL, SE)

1. Composition durcissable comprenant un éther polyglycidylique de la formule générale I 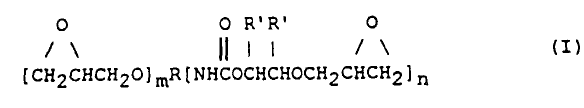 dans laquelle R représente un groupe de liaison aliphatique ou cycloaliphatique en C₁ à C₁₅, R' représente un atome d'hydrogène ou un radical alkyle en C₁ à C₃, au moins l'un des symboles R' représentant un atome d'hydrogène, m est égal à 0, 1, 2 ou 3, n est égal à 1, 2, 3 ou 4 et m + n est égal à 2, 3 ou 4 et une résine à fonctions anhydride ou carboxyle.

2. Composition suivant la revendication 1, caractérisé en ce que ladite résine est une résine acrylique ou une résine de polyester.

## Revendications (Revendications pour l'(les) Etat(s) contractant(s) suivant(s): ES)

1. Procédé de préparation d'une composition durcissable, caractérisé en ce que l'on mélange un éther polyglycidylique de la formule générale I 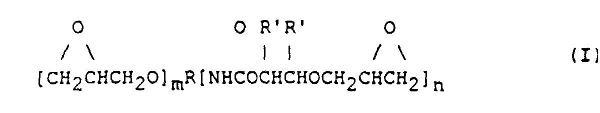 dans laquelle R représente un groupe de liaison aliphatique ou cycloaliphatique en C₁ à C₁₅, R' représente un atome d'hydrogène ou un radical alkyle en C₁ à C₃, au moins l'un des symboles R' représentant un atome d'hydrogène, m est égal à 0, 1, 2 ou 3, n est égal à 1, 2, 3 ou 4 et m + n est égal à 2, 3 ou 4 et une résine à fonctions anhydride ou carboxyle.

2. Procédé suivant la revendication 1, caractérisé en ce que la résine est une résine acrylique ou une résine de polyester.
